(19)

Europäisches
Patentamt

European
Patent Office

Office européen
des brevets

(11) **EP 3 553 526 A1**

(12) **EUROPEAN PATENT APPLICATION**

(43) Date of publication:
**16.10.2019 Bulletin 2019/42**

(51) Int Cl.:
***G01N 33/68*** *(2006.01)*

(21) Application number: **18166979.7**

(22) Date of filing: **12.04.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AE Eindhoven (NL)**

(72) Inventors:
- **KOOIJMAN, Gerben
  5656 AE Eindhoven (NL)**
- **VAN HARTSKAMP, Michael Alex
  5656 AE Eindhoven (NL)**
- **RMAILE, Amir Hussein
  5656 AE Eindhoven (NL)**

- **GLASSE, Carl
  5656 AE Eindhoven (NL)**
- **DE JAGER, Marinus Karel Johannes
  5656 AE Eindhoven (NL)**
- **PRESHAW, Philip
  5656 AE Eindhoven (NL)**
- **TAYLOR, John
  5656 AE Eindhoven (NL)**
- **CHAPPLE, Iain Leslie Campbell
  5656 AE Eindhoven (NL)**
- **GRANT, Melissa Mackay
  5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **METHODS, USES AND KITS FOR MONITORING OR PREDICTING RESPONSE TO PERIODONTAL DISEASE TREATMENT**

(57) Disclosed is an *in vitro* method for assessing or predicting the response of a human patient to treatment of periodontal disease. The method is based on the insight to determine biomarker proteins. Accordingly, in a sample of saliva of a patient, the concentrations are measured of certain protein combinations. One such combination is Hemoglobin subunit delta (Hb-delta) and Pyruvate kinase (PK). Another such combination is Keratin-4 (K-4) and at least two of Alpha-1-acid glycoprotein (A1AGP), Pyruvate Kinase (PK) and Matrix metalloproteinase-8 (MMP-8). A third combination is Alpha-1-acid glycoprotein (A1AGP), Pyruvate Kinase (PK) and S100 calcium binding protein A8 (S100A8). Based on the concentrations as measured, at least one value is determined reflecting the joint concentrations for said proteins. This at least one value may indicate the probability that human patient has been or will be successfully treated for the periodontitis. The at least one value can be compared with at least one threshold value reflecting in the same manner the joint concentrations associated with successful treatment of periodontitis. The comparison allows assessing whether the testing value is indicative of the periodontal treatment status in said patient.

**EP 3 553 526 A1**

**Description**

FIELD OF THE INVENTION

The invention is in the field of oral care, and pertains to saliva-based evaluation of response to treatment for periodontal disease. Particularly, the invention pertains to a kit, use and methods for assessing or predicting the response to treatment of a patient suffering from periodontal disease.

BACKGROUND OF THE INVENTION

Gum inflammation, or gingivitis, is a non-destructive periodontal disease caused mainly by the adherence of dental bacterial biofilms, or dental plaque, to the tooth surface. If not detected and treated, the reversible gingivitis usually leads to the inflammation of the tissues surrounding the tooth (i.e. periodontal tissues), a condition defined as periodontitis, which is irreversible and causes tissue destruction and alveolar bone loss, and ultimately results in the loss of teeth. During the progression of gum disease, there are usually clinical signs and symptoms associated with it, such as the swelling of the gums, the change in color from pink to dark red, the bleeding of the gums, bad breath, and the gums becoming more tender or painful to touch.

Periodontitis is a chronic multifactorial inflammatory disease caused by oral microorganisms and characterized by progressive destruction of the hard (bone) and soft (periodontal ligament) tissues, ultimately leading to tooth mobility and loss. This is to be distinguished from gingivitis which is a reversible infection and inflammation of the gum tissues. Inflammatory periodontitis is one of the most prevalent chronic human diseases and a major cause of adult tooth loss. In addition to the substantial negative impact of periodontitis on oral health, there is also mounting evidence that periodontitis has systemic consequences and that it is a risk factor for several systemic diseases, including heart diseases (e.g. atherosclerosis, stroke), diabetes, pregnancy complications, rheumatoid arthritis and respiratory infections.

Early and accurate diagnosis of periodontal disease, thus, is important from both an oral and overall health perspective. Furthermore, it would be desirable to be able to determine accurately whether a treatment of periodontal disease is, or is likely to be, effective in a patient. In particular, it would be desirable to be able to predict whether a treatment for periodontal disease is likely to be effective before it is provided to the patient or at an early stage of treatment.

Periodontal diseases are still poorly diagnosed in general dental practice, resulting in relatively low rates of therapeutic intervention and significant amounts of untreated cases. Current diagnosis relies on imprecise, subjective clinical examination of oral tissue condition (color, swelling, extent of bleeding on probing, probing pocket depth; and bone loss from oral x-rays) by dental professionals. These conventional methods are time consuming, and some of the techniques used (pocket-depth, x-ray) reflect historic events, such as past disease activity, rather than current disease activity or susceptibility to further disease.

Similarly, for periodontal patients receiving treatment, the response to treatment currently has to be clinically assessed via these conventional methods post-treatment, resulting in high cost and potentially inaccurate monitoring of the patient's condition. Furthermore, the possibility of predicting treatment response can be of large value, as treatment strategy may be adopted accordingly. Thereby it is desirable to measure current disease activity, a subject's susceptibility to further periodontal disease, and what treatment is likely to be successful in that patient. Hence, more objective, faster, accurate, easier-to-use diagnostics - ideally with predictive value - and which preferably may also be performed by non-specialists, are desirable.

Saliva or oral fluids have long been advocated as a diagnostic fluid for oral and general diseases, and with the advent of miniaturized biosensors, also referred to as lab-on-a-chip, point of care diagnostics for rapid chair-side testing have gained greater scientific and clinical interest. Especially for periodontal disease detection, inflammatory biomarkers associated with tissue inflammation and breakdown may easily end up in saliva due to proximity, suggesting saliva has strong potential for periodontal disease detection. Indeed, this area thus has gained significant interest and encouraging results have been presented. For example, Ramseier et al (J Periodontol. 2009 Mar;80(3):436-46) identified host- and bacterially derived biomarkers correlated with periodontal disease. However, no definite test has emerged yet.

Biomarkers represent biological indicators that underpin clinical manifestations, and as such are objective measures by which to diagnose clinical outcomes of periodontal disease. Ultimately, proven biomarkers could be utilized to assess risk for future disease, to identify disease at the very earliest stages, to identify response to initial therapy, and to allow implementation of preventive strategies.

Previous limitations to the development of point-of-care tests for salivary biomarkers included a lack of technologies that were adaptable to chair-side applications and an inability to analyze multiple biomarkers in individual samples. Also the selection of which multiple biomarkers to include in such a test has not been adequately addressed in the literature nor implemented in practical tests.

Moreover, periodontitis can manifest itself across the entire spectrum of severity ranging from mild to advanced forms of the disease. To assess the severity of the condition easily, dentists often classify patients suffering from periodontitis into two groups - those suffering from *mild* periodontitis, and those suffering from *advanced* periodontitis. The available methods of making such an assessment, however, involve a labor intensive process that a dentist will not perform routinely on every patient and/or on every visit, and that is impossible to perform by a consumer (self-diagnosis).

It would be desired to provide a simpler process, and particularly a process that requires only that a small saliva sample is taken from a patient, and possibly by the patient him- or herself. It is desired that such a sample be entered into an *in vitro* diagnostic device, which will allow, based on measurement, a classification of the saliva sample such that it can return an indication of the likelihood that periodontal disease in the patient is being, or is likely to be, effectively treated.

## SUMMARY OF THE INVENTION

In order to better address the foregoing desires, the invention, in one aspect, concerns an *in vitro* method for assessing or predicting the response of a human patient to treatment of periodontal disease, the method comprising detecting, in a sample of saliva from said human patient suffering from periodontal disease, the concentrations of at least the proteins:

(i) Hemoglobin subunit delta (Hb-delta) and Pyruvate kinase (PK); or
(ii) Keratin-4 (K-4) and at least two of Alpha-1-acid glycoprotein (A1AGP), Pyruvate Kinase (PK) and Matrix metalloproteinase-8 (MMP-8); or
(iii) Alpha-1-acid glycoprotein (A1AGP), Pyruvate Kinase (PK) and S100 calcium binding protein A8 (S100A8);

determining at least one testing value reflecting the joint concentrations determined for said proteins, and comparing said testing value with a threshold value reflecting in the same manner the joint concentrations associated with successful treatment of periodontal disease, so as to assess whether the testing value is indicative for successful treatment of periodontal disease in said patient.

In another aspect, the invention presents the use of the proteins identified above in a saliva sample of a human patient, as biomarkers for assessing whether the patient will respond, or has responded to, periodontal disease treatment.

Optionally, the age of the patient is also used as a biomarker.

In a further aspect, the invention resides in a system for assessing or predicting the response of a human patient to treatment of periodontal disease, the system comprising:

- detection means able and adapted to detect in a sample of saliva of the human patient the proteins identified in the first aspect; and
- a processor able and adapted to determine from the determined concentrations of said proteins an indication whether the periodontal disease in the patient has been or will be successfully treated.

The system optionally contains a data connection to an interface, particularly a graphical user interface, capable of presenting information, preferably also capable of putting in information, said interface being either a part of the system or a remote interface.

Optionally one or more of the foregoing items, particularly the processor, are enabled to function "in the cloud", i.e., not on a fixed machine, but by means of an internet-based application.

In a still further aspect, the invention provides a kit for detecting at least two biomarkers for periodontal disease in a sample of saliva of a human patient, said kit comprising detection reagents for detecting:

Hemoglobin subunit delta (Hb-delta) and Pyruvate kinase (PK); or
Keratin-4 (K-4) and at least two of Alpha-1-acid glycoprotein (A1AGP), Pyruvate Kinase (PK) and Matrix metalloproteinase-8 (MMP-8); or
Alpha-1-acid glycoprotein (A1AGP), Pyruvate Kinase (PK) and S100 calcium binding protein A8 (S100A8).

Typically, two or more, three or four detection reagents are used, each of which binds a different biomarker. In one embodiment, a first detection reagent is for detecting Hb-delta and a second detection reagent is for detecting PK. In another embodiment, a first detection reagent is for detecting K-4, a second detection reagent is for detecting A1AGP, and a third detection reagent is for detecting PK. In another embodiment, a first detection reagent is for detecting K-4, a second detection reagent is for detecting A1AGP, and a third detection reagent is for detecting MMP-8. In another embodiment, a first detection reagent is for detecting K-4, a second detection reagent is for detecting MMP-8, and a third detection reagent is for detecting PK. In another embodiment, a first detection reagent is for detecting A1AGP, a second detecting reagent is for detecting PK, and a third detecting reagent is for detecting S100A8. In a further embodiment, a first detection reagent is for detecting Keratin-4 (K-4), a second detection reagent is for detecting Alpha-1-acid glycoprotein (A1AGP), a third detection reagent is for detecting Pyruvate Kinase (PK) and a fourth detection reagent is for detecting Matrix metalloproteinase-8 (MMP-8).

In yet another aspect, the invention provides an *in vitro* method for determining a change in status of periodontal disease due to treatment of the disease in a human patient over a time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the proteins:

Hemoglobin subunit delta (Hb-delta) and Pyruvate kinase (PK); or

Keratin-4 (K-4) and at least two of Alpha-1-acid glycoprotein (A1AGP), Pyruvate Kinase (PK) and Matrix metalloproteinase-8 (MMP-8); or

Alpha-1-acid glycoprotein (A1AGP), Pyruvate Kinase (PK) and S100 calcium binding protein A8 (S100A8);

and comparing the concentrations, whereby a difference in any one, two, three, four or more of the concentrations, reflects a change in status.

In a further aspect, the invention provides a method of determining whether a human patient has been, is being, or will be successfully treated for periodontal disease, comprising detecting in a sample of saliva of the human patient the proteins identified in the first aspect above, and assessing whether the human patient has been or will be successfully treated for periodontal disease on the basis of the concentrations of said proteins in said sample. Optionally, the method comprises the further step of treating the periodontitis in the patient.

In yet a further aspect, the invention provides a method of detecting the proteins identified in the first aspect above in a human patient, comprising:

(a) obtaining a saliva sample from a human patient; and

(b) detecting whether the proteins are present in the sample by contacting the sample with two or more detecting reagents for binding said proteins and detecting binding between each protein and the two or more detecting reagents. Typically, there are at least first and second, and sometimes third and fourth, detection reagents as set out elsewhere herein.

BRIEF DESCRIPTION OF THE DRAWINGS

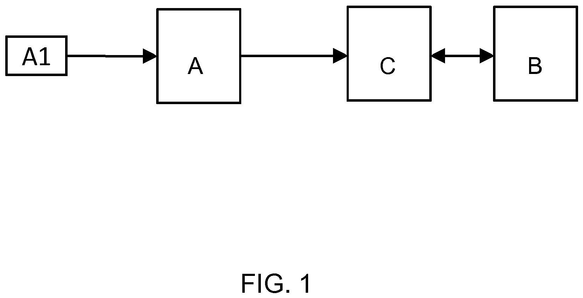

Fig. 1 schematically represents a system for use in the method as described in this disclosure.

DETAILED DESCRIPTION OF THE INVENTION

In a general sense, the invention is based on the judicious insight that certain combinations of protein biomarkers in a sample of saliva of a human patient, can be used for assessing or predicting the response to treatment of periodontal disease in a patient. The biomarker combinations in saliva are able to differentiate a successful response to periodontitis treatment from an unsuccessful response to periodontitis treatment. This insight is based at least in part on the finding that the usefulness of a biomarker for diagnosing periodontal disease (e.g. before treatment) does not necessarily mean that it is also useful for monitoring the treatment of that periodontal disease. The current disclosure presents clinical definitions of various levels of treatment response, and identifies the salivary protein marker combinations that enable assessment or prediction of treatment response from measurement of their concentrations post-treatment, or (for prediction) pre-treatment.

The biomarker proteins are Hemoglobin subunit delta (Hb-delta), Pyruvate kinase (PK), Keratin-4 (K-4), Alpha-1-acid glycoprotein (A1AGP), Matrix metalloproteinase-8 (MMP-8), and S100 calcium binding protein A8 (S100A8). The following combinations of these proteins are used to monitor or predict the treatment of periodontal disease according to the invention:

i. Hemoglobin subunit delta (Hb-delta) and Pyruvate kinase (PK); or

ii. Keratin-4 (K-4) and at least two of Alpha-1-acid glycoprotein (A1AGP), Pyruvate Kinase (PK) and Matrix metalloproteinase-8 (MMP-8); or

iii. Alpha-1-acid glycoprotein (A1AGP), Pyruvate Kinase (PK) and S100 calcium binding protein A8 (S100A8).

The subject's age may optionally be included as an additional marker.

In one embodiment, the method assesses the response of a human patient previously diagnosed as having periodontitis and that has received treatment for that periodontitis. Typically, the concentrations of the proteins Hemoglobin subunit delta (Hb-delta) and Pyruvate kinase (PK) can be detected in this embodiment.

In another embodiment, the method predicts the response of a human patient to a treatment for periodontitis.

This may be when the treatment has not yet been administered to the patient. Alternatively, a treatment may have been administered recently to the patient and it is desired to know at an early stage whether it is likely to be effective. Typically, the treatment is first administered at least about one week, about two weeks or about three weeks, for example at least about 7 days, at least about 14 days or at least about 21 days prior to assessment of the patient using the method of the invention. The treatment may begin between about one week and between about one month prior to assessment. The patient may optionally be assessed at two, three or four week intervals after first treatment, for example at weeks 3, 6 and 9 after treatment, or weeks 4, 8 and 12 after treatment. In these predictive embodiments, the concentrations of the proteins Keratin-4 (K-4) and at least two of Alpha-1-acid glycoprotein (A1AGP), Pyruvate Kinase (PK) and Matrix metalloproteinase-8 (MMP-8) may be detected. Alternatively, the concentrations of the proteins Alpha-1-acid glycoprotein (A1AGP), Pyruvate Kinase (PK) and S100 calcium binding protein A8 (S100A8) may be detected.

Haemoglobin (Hb) is the iron-containing oxygen-transport metalloprotein in the red blood cells of nearly all vertebrates as well as the tissues of some invertebrates. Haemoglobin subunit delta (also known as delta globin, HBD, $\delta$-globin, and haemoglobin delta) is a globin protein, which along with alpha globin (HBA), makes up the less common form of haemoglobin in adult humans, the HbA-2. Hb-delta is typically 147 amino acids long and has a molecular weight of 16,055 Da. Adult human HbA-2 is a heterotetramer consisting of two alpha chains and two delta chains. Hb-delta is encoded by the HBD gene on human chromosome 11.

Pyruvate kinase catalyses the final step of glycolysis. There are four tissue-specific isozymes of Pyruvate Kinase, each having particular kinetic properties needed for different tissues.

Keratin-4 (K4), also known as cytoskeletal Keratin 4 (CYK4) or cytokeratin-4 (CK-4) is a protein that in humans is encoded by the KRT4 gene. It is a member of the keratin gene family. The type II cytokeratins consist of basic or neutral proteins which are arranged in pairs of heterotypic keratin chains coexpressed during differentiation of simple and stratified epithelial tissues. The type II cytokeratin CK4 is specifically expressed in differentiated layers of the mucosal and esophageal epithelia with family member KRT13. Mutations in these genes have been associated with White Sponge Nevus, characterized by oral, esophageal, and anal leukoplakia. The type II cytokeratins are clustered in a region of chromosome 12q12-q13.

Alpha-1-acid glycoprotein (A1AGP) is a plasma alpha-globulin glycoprotein synthesized primarily by the liver. It is also sometimes known as Orosomucoid. It functions as a transport protein in the blood acts as a carrier of basic and neutrally charged lipohillic compounds. It is also believed to regulate the interaction between blood cells and endothelial cells.

MMPs are a family of enzymes that are responsible for the degradation of extracellular matrix components such as collagen, proteoglycans, laminin, elastin, and fibronectin. They play a central role in the periodontal ligament (PDL) remodelling, both in physiological and pathological conditions. MMP-8, also known as neutrophil collagenase or PMNL collagenase (MNL-CL), is a collagen protease enzyme which is present in the connective tissue of most mammals.

S100 calcium binding protein A8 is a calcium- and zinc-binding protein which plays a prominent role in the regulation of inflammatory processes and immune response. It can induce neutrophil chemotaxis and adhesion.

The proteins mentioned above are known in the art. The skilled person is aware of their structure, and of methods to detect them in an aqueous sample, such as a saliva sample. Hereinafter the following protein biomarker combinations are collectively referred to as "the biomarker panels of the invention":

    i. Hemoglobin subunit delta (Hb-delta) and Pyruvate kinase (PK); or
    ii. Keratin-4 (K-4) and at least two of Alpha-1-acid glycoprotein (A1AGP), Pyruvate Kinase (PK) and Matrix metalloproteinase-8 (MMP-8); or
    iii. Alpha-1-acid glycoprotein (A1AGP), Pyruvate Kinase (PK) and S100 calcium binding protein A8 (S100A8).

A biomarker panel of the invention, in one embodiment, may consist of the protein biomarkers identified. Preferably, a biomarker panel of the invention consists of not more than four of the protein biomarkers identified in the invention. In addition to the biomarker panels of the invention, other biomarkers and or data, such as demographic data (e.g., age, sex) can be included in a set of data applied for the determination of the type of periodontitis. Additional protein biomarkers include Profilin, Haemoglobin-delta, Matrix metalloprotease-9, and Hepatocyte Growth Factor, each of which is present in an effective biomarker panel in the Example.

When other biomarkers are optionally included, the total number of biomarkers (i.e. the biomarker panel of the invention plus other biomarkers) is typically 4, 5 or 6.

However, a desirable advantage of the present invention is that the classification of periodontal disease in a patient can be determined by measuring preferably not more than four biomarkers, and more preferably measuring only three biomarkers, or even only two biomarkers in some embodiments. Certain preferred biomarker panels consist of:

    (i) Hemoglobin subunit delta (Hb-delta) and Pyruvate kinase (PK); or
    (ii) Alpha-1-acid glycoprotein (A1AGP), Pyruvate Kinase (PK) and S100 calcium binding protein A8 (S100A8); or

(iii) Keratin-4 (K-4), Alpha-1-acid glycoprotein (A1AGP), and Pyruvate Kinase (PK); or
(iv) Keratin-4 (K-4) Alpha-1-acid glycoprotein (A1AGP), and Matrix metalloproteinase-8 (MMP-8); or
(v) Keratin-4 (K-4), Pyruvate Kinase (PK), and Matrix metalloproteinase-8 (MMP-8); or
(vi) Keratin-4 (K-4), Alpha-1-acid glycoprotein (A1AGP), Pyruvate Kinase (PK) and Matrix metalloproteinase-8 (MMP-8).

Particularly, the determination does not need to involve the use of other data, which advantageously provides a simple and straightforward diagnostic test. The biomarker panels identified herein allow the detection of a successful outcome of periodontitis treatment.

The method, as desired, requires only that a small saliva sample, e.g. a dropsize, is taken from the subject. The size of the sample will typically range of from 0.1 μl to 2 ml, such as 1-2 ml, whereby smaller amounts, e.g., 0. 1 to 100 μl can be used for *in vitro* device processing, and whereby taking a larger sample, such as up to 20 ml, such as 7.5 to 17 ml, is also possible.

This sample is entered into an *in vitro* diagnostic device, which measures the concentrations of the proteins involved, and which returns an outcome, classifying the subject on the basis of a likelihood of successful periodontal disease treatment.

The ease of use of this invention will make it possible to test the majority of dental patients having periodontal disease, on a regular basis (e.g. as part of a regular dental check or even at home). This allows, *inter alia,* detecting whether treatment of periodontitis is or will be successful, and thus enables a more informed approach to treatment of the periodontitis, whereby a successful treatment can be continued and an unsuccessful treatment ceased or not begun. The ability to assess that the therapy is successful is beneficial to confirm, for example, that the patient's current treatment is satisfactory. Particularly, the method is also suitable for self-diagnosis, whereby the steps of taking the sample and entering it into a device can be conducted by the patient him- or herself.

The patient may typically be known to have periodontal disease when the invention is carried out. The patient may typically be known to have periodontitis when the invention is carried out. The periodontitis may be mild or advanced. In certain embodiments therefore, the method is for assessing whether a human patient, known to have periodontitis, is being (or will be) successfully treated for that condition.

The therapy that is assessed as successful or not may be any therapy for periodontal disease. Therapeutic agents and dental procedures, or a combination of therapeutic agents and dental procedures, are known and may be used. Known therapeutic agents include the administration of antimicrobial-containing agents such as a mouthwash, chip, gel or microsphere. A typical antimicrobial agent for use in treating gingivitis and periodontitis is chlorhexidine. Other therapeutic agents include antibiotics, typically orally-administered antibiotics, and enzyme suppressants such as doxycycline. Known non-surgical therapeutic procedures include: root surface instrumentation to disrupt subgingival plaque biofilms; scaling and root planing (SRP); and interproximal cleaning. Known surgical procedures include surgical pocket reduction, flap surgery, gum grafts or bone grafts. The therapy will preferably be a therapeutic agent such as an anti-microbial agent, typically an anti-microbial mouthwash.

The need for, and number of, optional visits to a dentist or hygienist for therapy can vary, according to clinical need and patient preference. An initial phase of clinical treatment is typically completed in a fewer number of longer appointments. In this embodiment, patients may attend a dental clinic for 2 appointments of approximately 1-2 hours each for the initial therapy. Following this, frequent recall within the early post-treatment period can check on healing, motivate patients, reinforce oral hygiene, and provide prophylaxis for removal of reforming plaque. This is typically achieved with short (e.g. 15-30 min) appointments at 2-4 week intervals for the next 1-2 months after the initial treatment period.

Accordingly, in certain embodiments the treatment phase may include 1-2 clinical appointments for root surface instrumentation. Following this, patients may return to the dentist or hygienist approximately 3, 6 and 9 weeks later for recall appointments (prophylaxis, motivation, reinforcement of oral hygiene). Within this schedule, treatment protocols can be implemented according to the clinical needs of the individual.

In certain embodiments, the assessment or prediction method of the invention may be carried out at any clinical visit subsequent to the initial therapy.

A method of the invention typically comprises detecting the aforementioned at least two proteins making up a biomarker panel of the invention, and optional further biomarker proteins, by using one or more detection reagents.

Typically, a treatment can be considered successful when inflammation levels are significantly lowered, whereas pocket depth may remain relatively high (i.e. compared to healthy individuals or gingivitis patients). Therefore, application of a disease classification (e.g. discriminating between health, gingivitis, mild periodontitis, and advanced periodontitis) based on salivary protein markers of disease *per se,* to patients post-treatment may not be suitable to assess treatment response. The biomarker proteins of the invention overcome this problem and are able to determine the response to treatment, specifically.

The "saliva" that is tested according to the invention may be undiluted saliva, which may be obtained by spitting or swabbing, or diluted saliva, which may be obtained by rinsing the mouth with a fluid. Diluted saliva may be obtained by the patient rinsing or swilling their mouth for a few seconds with sterile water (for example 5ml or 10ml) or other suitable fluid, and spitting into a container. Diluted saliva may sometimes be referred to as an oral rinse fluid.

By "detecting" is meant measuring, quantifying, scoring, or assaying the concentration of the biomarker proteins. Methods of evaluating biological compounds, including biomarker proteins, are known in the art. It is recognized that methods of detecting a protein biomarker include direct measurements and indirect measurements. One skilled in the art will be able to select an appropriate method of assaying a particular biomarker protein.

The term "concentration" with respect to the protein biomarkers is to be given its usual meaning, namely the abundance of the protein in a volume. Protein concentration is typically measured in mass per volume, most typically mg/ml or $\mu$g/ml, but sometimes as low as pg/ml. An alternative measure is Molarity (or Molar concentration), mol/L or "M". The concentration can be determined by detecting the amount of protein in a sample of known, determined or pre-determined volume.

An alternative to determining the concentration is to determine the absolute amount of the protein biomarker in the sample, or determining the mass-fraction of the biomarker in the sample, for example the amount of the biomarker relative to the total of all other proteins in the sample.

A "detection reagent" is an agent or compound that specifically (or selectively) binds to, interacts with or detects the protein biomarker of interest. Such detection reagents may include, but are not limited to, an antibody, polyclonal antibody, or monoclonal antibody that preferentially binds the protein biomarker.

The term "periodontal disease" refers to gingivitis and periodontitis (mild and advanced). A patient that is free from periodontal disease is said to be healthy.

The phrase "specifically (or selectively) binds" or "specifically (or selectively) immunoreactive with", when referring to a detection reagent, refers to a binding reaction that is determinative of the presence of the protein biomarker in a heterogeneous population of proteins and other biologics. Thus, under designated immunoassay conditions, the specified detection reagent (e.g. antibody) binds to a particular protein at least two times the background and does not substantially bind in a significant amount to other proteins present in the sample. Specific binding under such conditions may require an antibody that is selected for its specificity for a particular protein. A variety of immunoassay formats may be used to select antibodies specifically immunoreactive with a particular protein. For example, solid-phase ELISA immunoassays (enzyme linked immunosorbent assay) are routinely used to select antibodies specifically immunoreactive with a protein (*see, e.g.,* Harlow & Lane, Antibodies, A Laboratory Manual (1988), for a description of immunoassay formats and conditions that can be used to determine specific immunoreactivity). Typically a specific or selective reaction will be at least twice the background signal or noise and more typically more than 10 to 100 times the background.

"Antibody" refers to a polypeptide ligand substantially encoded by an immunoglobulin gene or immunoglobulin genes, or fragments thereof, which specifically binds and recognizes an epitope (e.g., an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, e.g., as intact immunoglobulins or as a number of well characterized fragments produced by digestion with various peptidases. This includes, e.g., Fab' and F(ab)'2 fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH1, CH2 and CH3, but does not include the heavy chain variable region. The antibody may be a bispecific antibody, e.g. an antibody that has a first variable region that specifically binds to a first antigen and a second variable region that specifically binds to a second, different, antigen. Use of at least one bispecific antibody can reduce the number of detection reagents needed.

Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. Specificity may also be referred to as the true negative rate.

The biomarker protein(s) of the invention can be detected in a sample by any means. Preferred methods for biomarker detection are antibody-based assays, protein array assays, mass spectrometry (MS) based assays, and (near) infrared spectroscopy based assays. For example, immunoassays, include but are not limited to competitive and non-competitive assay systems using techniques such as Western blots, radioimmunoassays, ELISA, "sandwich" immunoassays, immunoprecipitation assays, precipitin reactions, gel diffusion precipitin reactions, immunodiffusion assays, fluorescent immunoassays and the like. Such assays are routine and well known in the art. Exemplary immunoassays are described briefly below (but are not intended by way of limitation).

Immunoprecipitation protocols generally comprise lysing a population of cells in a lysis buffer such as RIPA buffer (1% NP-40 or Triton X-100, 1 % sodium deoxycholate, 0.1% SDS, 0.15 M NaCl, 0.01 M sodium phosphate at pH 7.2, 1 % Trasylol) supplemented with protein phosphatase and/or protease inhibitors (e.g., EDTA, PMSF, aprotinin, sodium vanadate), adding an antibody of interest to the cell lysate, incubating for a period of time (e.g., 1-4 hours) at 4°C, adding protein A and/or protein G sepharose beads to the cell lysate, incubating for about an hour or more at 4°C, washing the beads in lysis buffer and resuspending the beads in SDS/sample buffer. The ability of the antibody to immunoprecipitate a particular antigen can be assessed by, e.g., western blot analysis. One of skill in the art would be knowledgeable as to the parameters that can be modified to increase the binding of the antibody to an antigen and decrease the background (e.g., pre-clearing the cell lysate with Sepharose beads).

Western blot analysis generally comprises preparing protein samples, electrophoresis of the protein samples in a polyacrylamide gel (e.g., 8%-20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, e.g., an anti-human antibody) conjugated to an enzymatic substrate (e.g., horseradish peroxidase or alkaline phosphatase) or radioactive molecule (e.g., 32P or 125I) diluted in blocking buffer, washing the membrane in wash buffer, and detecting the presence of the antigen. One of skill in the art would be knowledgeable as to the parameters that can be modified to increase the signal detected and to reduce the background noise.

ELISAs typically comprise preparing antigen (i.e. the biomarker protein of interest or fragment thereof), coating the well of a 96- well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art.

Since multiple markers are used, a threshold can be determined on the basis of the joint concentrations of these biomarkers. This threshold determines whether a patient is classified as having been successfully treated or not. The invention reflects the insight that successful response to treatment of periodontitis can be distinguished from unsuccessful response to treatment of periodontitis with sufficient accuracy based on a measurement of the combination of biomarkers as indicated above.

This insight supports another aspect, the invention, which is the use of the protein combinations of the invention, as biomarkers in a saliva sample of a human patient, for assessing whether a periodontitis treatment is or will be successful in the patient. For the avoidance of doubt, the protein combinations of this aspect are:

Hemoglobin subunit delta (Hb-delta) and Pyruvate kinase (PK); or
Keratin-4 (K-4) and at least two of Alpha-1-acid glycoprotein (A1AGP), Pyruvate Kinase (PK) and Matrix metalloproteinase-8 (MMP-8); or
Alpha-1-acid glycoprotein (A1AGP), Pyruvate Kinase (PK) and S100 calcium binding protein A8 (S100A8).

This use can be implemented in a method as substantially described hereinbefore and hereinafter.

The method of the invention comprises determining at least one testing value reflecting the joint concentrations measured for said proteins. A joint concentration value can be any value obtained by input of the concentrations as determined and an arithmetic operation of these values. This can, e.g., be a simple addition of the concentrations. It can also involve multiplying each concentration with a factor reflecting a desired weight of these concentrations, and then adding up the results. It can also involve multiplying the concentrations with each other, or any combination of multiplication, division, subtraction, exponentiation, and addition. It can further involve raising concentrations to some power. Optionally, the testing value reflects the joint concentrations determined for said proteins in combination with the age of the subject.

The resulting joint concentration value may be compared with one or more threshold values reflecting in the same manner the joint concentrations associated with the successful treatment of periodontitis. The comparison allows assessing whether the testing value is indicative of the presence of successful treatment in the patients whose saliva is subjected to the test.

The threshold value can, e.g., be the joint concentration value, obtained in the same manner on the basis of the concentrations determined for the same proteins in a reference sample associated with the successful treatment of periodontitis, e.g. in a patient that was previously diagnosed with periodontitis and has been treated to reduce the severity of the disease. Typically, thereby a value reflecting the same or higher joint concentration is indicative of the successful treatment of periodontitis in a tested patient. Analogously, a value reflecting a lower joint concentration in the saliva of a tested periodontitis patient, indicates that the periodontitis has not been successfully treated. However, it will be understood that it is also possible to calculate a threshold value (e.g. by using a negative multiplier) such that a testing value indicating successful; treatment would be below the threshold, and a testing value indicating unsuccessful treatment, would be above the threshold.

The threshold value can also be determined on the basis of measuring the concentrations of the present biomarker proteins in a set of samples, including patients known to have been successfully treated and patients that were not successfully treated. Thereby the measured concentration values can be subjected to statistical analysis, possibly including machine learning methods, allowing to discriminate, with the desired sensitivity and specificity, patients classified as having been successfully treated or not. Therefrom, the desired threshold value(s) can be obtained. On the basis of this threshold value, a sample to be tested can be subjected to the same concentration measurement, and the concentration values are then processed, in the same manner in which the threshold value(s) is obtained, so as to determine a joint concentration value that can be compared with the threshold, thus allowing the tested sample to be classified as "yes" or "no" for successful treatment.

In an interesting embodiment, the joint concentration value is obtained in the form of a score as follows. A numerical value (protein concentration values in e.g. ng/ml) is assigned to each measurement, and these values are used in a linear or non-linear combination to calculate a score between zero and one. In the event that the threshold value is determined on the basis of a set of subjects as mentioned above, the score between 0 and 1 is typically calculated with the sigmoid function that takes the joint concentration as input (as shown further on).

When the score exceeds a certain threshold, the method indicates successful treatment of periodontitis. The threshold may be chosen based on the desired sensitivity and specificity.

Clinical definitions as acknowledged in the art are based on the following:

*Gingival Index (GI)*

A full mouth gingival index will be recorded based on the Lobene Modified Gingival Index (MGI) rated on a scale of 0 to 4, where:

- 0 = absence of inflammation,
- 1 = mild inflammation; slight change in color little change in texture of any portion of but not the entire margin or papillary gingival unit,
- 2 = mild inflammation; but involving entire margin or papillary unit,
- 3 = moderate inflammation; glazing, redness, oedema and/or hypertrophy of margin or papillary unit,
- 4 = severe inflammation; marked redness, oedema and/or hypertrophy of marginal or papillary gingival unit, spontaneous bleeding, congestion, or ulceration].

*Probing depths (PD)*

Probing depths will be recorded to the nearest mm using a manual UNC-15 periodontal probe. Probing depth is the distance from the probe tip (assumed to be at the base of the pocket) to the free gingival margin.

*Gingival recession (REC)*

Gingival recession will be recorded to the nearest mm using a manual UNC-15 periodontal probe. Gingival recession is the distance from the free gingival margin to the cemento-enamel junction. Gingival recession will be indicated as a positive number and gingival overgrowth will be indicated as a negative number.

*Clinical attachment loss (CAL)*

Clinical attachment loss will be calculated as the sum of probing depth + recession at each site.

*Bleeding on probing (BOP)*

Following probing, each site will be assessed for bleeding on probing, if bleeding occurs within 30s of probing, a score of 1 will be assigned for the site, otherwise a score of 0 will be assigned.

The resulting subject group (patient group) definition is as follows:

- Healthy group (H): PD ≤ 3 mm in all sites (but would allow up to four 4 mm pockets at distal of last standing molars), no sites with interproximal attachment loss, GI of ≥ 2.0 in ≤ 10% sites, %BOP scores ≤ 10%;

- Gingivitis group (G): GI ≥ 3.0 in > 30% of sites, no sites with interproximal attachment loss, no sites with PD > 4 mm , %BOP scores > 10%;
- Mild-moderate periodontitis group (MP): interproximal PD of 5-7 mm, (equating to approximately 2-4 mm CAL) at ≥ 8 teeth, %BOP scores > 30%;
- Advanced periodontitis group (AP): interproximal PD of ≥ 7 mm, (equating to approximately ≥ 5 mm CAL) at ≥ 12 teeth, %BOP scores > 30%.

In an embodiment, the method of the invention makes use of a system as represented schematically in Fig. 1. The system can be a single apparatus having various device components (units) integrated therein. The system can also have its various components, or some of these components, as separate apparatuses. The components shown in Fig. 1 are a measurement device (A), a graphical user interface (B) and a computer processing unit (C).

As mentioned above, the system of the invention comprises a data connection to an interface, whereby the interface itself can be a part of the system or can be a remote interface. The latter refers to the possibility to use a different apparatus, preferably a handheld apparatus such as a smartphone or a tablet computer, for providing the actual interface. The data connection in such cases will preferably involve wireless data transfer such as by Wi-Fi or Bluetooth, or by other techniques or standards.

The measurement device (A) is configured to receive a saliva sample, for example by putting a drop of saliva on a cartridge (A1), which can be inserted into the device (A). The device can be an existing device that is capable to determine, from the same saliva sample, the concentrations of at least a biomarker protein combination of the invention, i.e.:

Hemoglobin subunit delta (Hb-delta) and Pyruvate kinase (PK); or
Keratin-4 (K-4) and at least two of Alpha-1-acid glycoprotein (A1AGP), Pyruvate Kinase (PK) and Matrix metalloproteinase-8 (MMP-8); or
Alpha-1-acid glycoprotein (A1AGP), Pyruvate Kinase (PK) and S100 calcium binding protein A8 (S100A8).

The measurement device (A) should be able to receive a saliva sample, for example by putting a drop of saliva on a cartridge (A1), which can be inserted into the device (A). The device may be an existing device that is capable to determine, from the same saliva sample, the concentrations of at least two of the protein biomarkers of the invention, for example HB-delta and PK, or A1AGP, PK and S100A8.

The processing unit (C) receives numerical values for the protein concentrations from part (A). The unit (C) is provided with software (typically embedded software) allowing it to calculate a score (S) between 0 and 1. The software further includes a numerical value for the threshold (T). If the calculated value (S) exceeds (T), unit (C) will output an indication (I) of 'successful treatment of periodontitis' to the GUI (B), otherwise it will output 'unsuccessful treatment of periodontitis'. A further embodiment may use the specific value of (S) to indicate the certainty with which the indication (I) is made. This can be 'directly', in the sense that e.g. a score S=0.8 would indicate 'successful treatment of periodontitis' with 80% certainty. Otherwise, it can be done e.g. through the definition of a range R1-R2, such that when R1<S<R2, the indication (I) will read 'inconclusive'.

A specific calculation of the scores can be implemented, e.g., by means of a logistic regression, employing the sigmoid function:

$$S = \frac{1}{1 + \exp(-(c_0 + \sum_{i=1}^{N} c_i B_i))}$$

With $N$ the number of proteins/biomarkers used. $c_0$, $c_1$, etc. are coefficients (numerical values) and $B_1, B_2$, etc. are the respective protein concentrations.

Determination of the coefficients $c_i$ can be done by a training procedure:

- Select N1 subjects who have a successful response to treatment of periodontitis (as identified by a dentist via the current criteria) and N2 subjects who have a unsuccessful response to treatment of periodontitis.
- Take a saliva sample from each subject and determine the protein concentrations of a combination of biomarkers as explained above (the saliva sample may be from a patient that has not yet received a treatment [for predicting a response], or a patient that has received a treatment [for assessing the response])
- Define the score S to be 1 for successful response, and 0 for unsuccessful response.
- Fit the sigmoid function to the scores and protein concentration values.

Note that alternatively any existing regression or machine learning method (e.g. linear regression, neural networks, support vector machines, etc.) may be used, where the score S is high for patients successfully responding to treatment and low for the patients not successfully responding to treatment.

In particular, such a procedure has been applied using a clinical study with subjects who showed either successful response to treatment or unsuccessful response to treatment of periodontitis, which was identified by clinical assessment by a dental professional. Here, patients who have a bleeding-on-probing percentage less or equal to 30 (BOP≤30%) after treatment, are considered to be high responders to treatment / to have successful treatment outcome. Patients with BOP>30% after treatment are considered to be low responders / have unsuccessful treatment response.

As used herein, "successful treatment" typically means bleeding-on-probing percentage less or equal to 30 (BOP≤30%) after treatment for periodontitis.

A multiclass classifier that indicates the "success" status may be used.

Performance of various biomarker combinations were evaluated by means of Leave-1-out cross validation, resulting in the preferred biomarker combinations of the invention.

With reference to the aforementioned system, the invention also provides, in a further aspect, a system for assessing whether a human patient has been or will be successfully treated for periodontitis, the system comprising:

- detection means able and adapted to detect in a sample of saliva of the human patient the proteins:

   Hemoglobin subunit delta (Hb-delta) and Pyruvate kinase (PK); or
   Keratin-4 (K-4) and at least two of Alpha-1-acid glycoprotein (A1AGP), Pyruvate Kinase (PK) and Matrix metalloproteinase-8 (MMP-8); or
   Alpha-1-acid glycoprotein (A1AGP), Pyruvate Kinase (PK) and S100 calcium binding protein A8 (S100A8).

As explained above, such means are known, and easily accessible to the skilled person. Typically, there is provided:

- a container for receiving an oral sample of a subject therein, the container provided with the detection means;
- a processor able and adapted to determine from the determined concentrations of said proteins an indication of yes/no successful response to treatment of periodontitis.

Optionally, the system comprises a user interface (or a data connection to remote interface), particularly a graphical user interface (GUI), capable of presenting information; a GUI is a type of user interface that allows users to interact with electronic devices through graphical icons and visual indicators such as secondary notation, instead of text-based user interfaces, typed command labels or text navigation (none of such interface types being excluded in the present invention); GUIs are generally known, and are used typically in handheld mobile devices such as MP3 players, portable media players, gaming devices, smartphones and smaller household, office and industrial controls; as said, the interface optionally can also be chosen so as to be capable of putting in information, such as, e.g., the age of the subject, sex, BMI (Body Mass Index).

The invention also provides, either separately or as part of the aforementioned system, a kit for detecting at least two biomarkers for periodontal disease in a sample of saliva of a human patient, said kit comprising one or more detection reagents for detecting:

   Haemoglobin subunit delta (Hb-delta) and Pyruvate kinase (PK); or
   Keratin-4 (K-4) and at least two of Alpha-1-acid glycoprotein (A1AGP), Pyruvate Kinase (PK) and Matrix metalloproteinase-8 (MMP-8); or
   Alpha-1-acid glycoprotein (A1AGP), Pyruvate Kinase (PK) and S100 calcium binding protein A8 (S100A8).

Typically, the kit comprises two or three detection reagents, each directed to a different biomarker. More typically, the detection reagents in the kit comprise or consist of:

   a first detection reagent for detecting Haemoglobin subunit delta (Hb-delta), and a second detection reagent for detecting Pyruvate Kinase (PK),
   a first detection reagent for detecting Keratin-4 (K-4), a second detection reagent for detecting Alpha-1-acid glycoprotein (A1AGP), and a third detection reagent for detecting Pyruvate Kinase (PK); or
   a first detection reagent for detecting Keratin-4 (K-4), a second detection reagent for detecting Alpha-1-acid glycoprotein (A1AGP), and a third detection reagent for detecting Matrix metalloproteinase-8 (MMP-8); or
   a first detection reagent for detecting Keratin-4 (K-4), a second detection reagent for detecting Pyruvate Kinase (PK), and a third detection reagent for detecting Matrix metalloproteinase-8 (MMP-8); or

a first detection reagent for detecting Keratin-4 (K-4), a second detection reagent for detecting Alpha-1-acid glycoprotein (A1AGP), a third detection reagent for detecting Pyruvate Kinase (PK) and a fourth detection reagent for detecting Matrix metalloproteinase-8 (MMP-8); or

a first detection reagent for detecting Alpha-1-acid glycoprotein (A1AGP), a second detection reagent for detecting Pyruvate Kinase (PK), and a third detection reagent for detecting S100 calcium binding protein A8 (S100A8).

As discussed above with reference to the method of the invention, the kit may comprise more detection reagents, such as for other proteins. In a preferred embodiment the detection reagents made available in the kit consist of the detection reagents for the detection of three proteins making up a biomarker panel of the invention, as mentioned.

Preferably said kits comprise a solid support, such as a chip, a microtiter plate or a bead or resin comprising said detection reagents. In some embodiments, the kits comprise mass spectrometry probes, such as ProteinChip™.

The kits may also provide washing solutions and/or detection reagents specific for either unbound detection reagent or for said biomarkers (sandwich type assay).

In an interesting aspect, the recognition of a biomarker panel of the invention is applied in monitoring the status of periodontal disease in a human patient, over a period of treatment. Accordingly, the invention also provides an *in vitro* method for determining a change in status of periodontal disease due to treatment of the disease in a human patient suffering from periodontitis over a therapeutic time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the proteins:

Hemoglobin subunit delta (Hb-delta) and Pyruvate kinase (PK); or

Keratin-4 (K-4) and at least two of Alpha-1-acid glycoprotein (A1AGP), Pyruvate Kinase (PK) and Matrix metalloproteinase-8 (MMP-8); or

Alpha-1-acid glycoprotein (A1AGP), Pyruvate Kinase (PK) and S100 calcium binding protein A8 (S100A8);

and comparing the concentrations, whereby a difference of preferably two, three or four concentrations, reflects a change in status. This difference can be reviewed as a difference in concentrations, thus allowing a direct comparison without first generating a number between 0 and 1, or any other classification. It will be understood that the measurements received at both points in time can also be processed in just the same manner as done when determining the patient status as above.

The invention also provides a method of diagnosing whether a human patient has been or will be successfully treated for periodontal disease, comprising detecting in the patient's saliva the presence of the proteins of the invention. The presence of successful therapy in the patient is assessed on the basis of the concentrations of said proteins in said sample. Optionally, the method of this aspect comprises the further step of treating the periodontal disease in the patient. This optional treatment step can comprise the administration of known therapeutic agents or dental procedures, or a combination of therapeutic agents and dental procedures. Known therapeutic agents include the administration of antimicrobial-containing agents such as a mouthwash, chip, gel or microsphere. A typical antimicrobial agent for use in treating gingivitis and periodontitis is chlorhexidine. Other therapeutic agents include antibiotics, typically orally-administered antibiotics, and enzyme suppressants such as doxycycline. Known non-surgical therapeutic procedures include scaling and root planing (SRP). Known surgical procedures include surgical pocket reduction, flap surgery, gum grafts or bone grafts.

The invention further provides a method of detecting the proteins of the invention in a patient, comprising:

(a) obtaining a saliva sample from a human patient; and

(b) detecting whether the proteins of the invention are present in the saliva sample by contacting the saliva sample with detection reagents for the proteins and detecting binding between each protein and detection reagent.

The invention will be further illustrated with reference to the following nonlimiting example.

**Example**

In a clinical study with 71 subjects undergoing repeated clinical visits at two independent clinical sites, who received treatment of periodontitis, of who:

- 17 had low/unsuccessful response
- 54 had high/successful response

we obtained Receiver-Operator-Characteristic Area-Under-the Curve values of >0.75 for detecting successful treatment outcome.

In statistics, a receiver operating characteristic curve, or ROC curve, is a graphical plot that illustrates the performance of a binary classifier system as its discrimination threshold is varied. The curve is created by plotting the true positive rate (TPR) against the false positive rate (FPR) at various threshold settings. The true-positive rate is also known as sensitivity, recall or *probability of detection* in machine learning. The false-positive rate is also known as the fall-out or *probability of false alarm* and can be calculated as (1 - specificity). The ROC curve is thus the sensitivity as a function of fall-out. In general, if the probability distributions for both detection and false alarm are known, the ROC curve can be generated by plotting for every value of the threshold, the value of the cumulative distribution function (area under the probability distribution from $-\infty$ to the discrimination threshold) of the detection probability on the y-axis, versus the value of the cumulative distribution function of the false-alarm probability on the x-axis. The accuracy of the test depends on how well the test separates the group being tested into those with and without the disease in question. Accuracy is measured by the area under the ROC curve. An area of 1 represents a perfect test; an area of 0.5 represents a worthless test. A guide for classifying the accuracy of a diagnostic test is the traditional academic point system:

- 0.90-1 = excellent (A)
- 0.80-0.90 = good (B)
- 0.70-0.80 = fair (C)
- 0.60-0.70 = poor (D)
- 0.50-0.60 = fail (F)

Various biomarker combinations were evaluated by means of logistic regression with leave-one-out cross validation (LOOCV), resulting in the biomarker combinations of the invention. It can be seen that the protein biomarker combinations of the invention have an AUC of >0.75 for detecting successful treatment outcome.

Based on the foregoing, in the results of the aforementioned clinical study, an ROC AUC value of above 0.75 is considered to represent a desirable accuracy for providing a test in accordance with the invention.

The protein biomarkers explored were:

- MMP8
- MMP9
- IL-1$\beta$
- HGF
- Free Light Chain (FLC) $\kappa$ (kappa)
- Free light chain (FLC) $\lambda$ (lambda)
- A1AGP
- Hb-beta
- Hb-delta
- Keratin 4
- Profilin
- Pyruvate Kinase
- S100A8
- S100A9

Furthermore, in the employed logistic regression we considered as additional predictors $\kappa+\lambda$, $\kappa-\lambda$, $\kappa/\lambda$.

Additionally we included age as predictor.

This yields a total number of 4204 possible non-redundant panels, having at most 4 protein biomarkers (panel having only age is not considered). Non-redundant here means that a panel including e.g. $\kappa+\lambda$ and $\kappa-\lambda$ as predictors is not considered, as in the logistic regression it gives the same result as the corresponding panel including $\kappa$ and $\lambda$ as predictors.

Note that not restricting the number of protein markers in a panel, yields a number of 98302 possible non-redundant panels (panel having only age is not considered) given the predictors mentioned above.

For assessment of treatment response based on post-treatment marker levels 5 panels were found with a performance of AUC LOOCV ~0.75, these are given in the following table, in which each biomarker panel is a preferred embodiment of the invention:

| MMP8 | IL1B | MMP9 | HGF | Age | $\kappa$ | $\lambda$ | $\kappa+\lambda$ | $\kappa/\lambda$ | $\kappa-\lambda$ | A1AGP | Hb-beta | Hb-delta | Keratin 4 | Profilin | Pyruvate Kinase | S100A8 | S100A9 | AUC LOOCV |
|---|---|---|---|---|---|---|---|---|---|---|---|---|---|---|---|---|---|---|
| | | | | | | | | | | | | X | | | X | | | 0.741 |
| | | | | | | | | | | | | X | X | | X | | | 0.748 |
| X | | | | | | | | | | | | X | X | | X | | | 0.748 |
| | X | | | | | | | | | | | X | X | | X | | | 0.749 |
| | | | X | | | | | | | | | X | X | | X | | | 0.744 |

For prediction of treatment success based on pre-treatment marker levels, the following 16 panels that provide a performance of AUC LOOCV>0.75 were found:

| MMP8 | IL1B | MMP9 | HGF | Age | κ | λ | κ+λ | κ/λ | κ-λ | A1AGP | Hb-beta | Hb-delta | Keratin 4 | Profilin | Pyruvate Kinase | S100A8 | S100A9 | AUC LOOCV |
|---|---|---|---|---|---|---|---|---|---|---|---|---|---|---|---|---|---|---|
| X |   |   |   |   |   |   |   |   |   | X |   |   | X |   |   |   |   | 0.751 |
| X | X |   |   |   |   |   |   |   |   | X |   |   | X |   |   |   |   | 0.765 |
| X |   |   |   |   |   |   |   |   |   | X | X |   | X |   |   |   |   | 0.764 |
| X |   |   |   | X |   |   |   |   |   | X | X |   | X |   |   |   |   | 0.751 |
| X |   |   |   |   |   |   |   |   |   |   |   |   | X | X |   |   |   | 0.761 |
| X |   | X |   |   |   |   |   |   |   |   |   |   | X | X |   |   |   | 0.768 |
| X |   | X | X |   |   |   |   |   |   |   |   |   | X | X |   |   |   | 0.761 |
| X |   |   |   |   |   |   |   |   |   | X |   |   | X | X |   |   |   | 0.816 |
|   | X |   |   |   |   |   |   |   |   | X |   |   | X | X |   |   |   | 0.769 |
| X |   |   | X |   |   |   |   |   |   | X |   |   | X | X |   |   |   | 0.796 |
|   | X | X |   |   |   |   |   |   |   | X |   |   | X | X |   |   |   | 0.762 |
| X |   |   |   |   |   |   |   |   |   |   | X |   | X | X |   |   |   | 0.768 |
| X |   |   | X |   |   |   |   |   |   |   | X |   | X | X |   |   |   | 0.762 |
| X |   |   |   |   |   |   |   |   |   |   |   |   | X | X | X |   |   | 0.750 |
| x |   |   |   |   |   |   |   |   |   | X |   |   |   |   | X | X |   | 0.769 |
|   |   | X |   |   |   |   |   |   |   | x |   |   |   |   | X | X |   | 0.751 |

Each of these panels is a preferred biomarker combination according to the invention.

While the invention has been illustrated and described in detail in the drawings and foregoing description, such illustration and description are to be considered illustrative or exemplary and not restrictive; the invention is not limited to the disclosed embodiments.

For example, it is possible to present detection reagents for different biomarkers in different units. Or, conveniently, a kit of the invention can comprise a fixed set of detection reagents for the protein biomarkers that are essential in an embodiments, i.e., K-4 in certain embodiments, and flexible modules comprising a detection reagent for either of the further biomarkers, e.g. A1AGP and/or PK.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain features of the invention are recited in mutually different dependent claims does not indicate that a combination of these features cannot be used to advantage. Any reference signs in the claims should not be construed as limiting the scope.

In sum, we hereby disclose an *in vitro* method for assessing or predicting the response of a human patient to treatment of periodontal disease. The method is based on the insight to determine a selection of as few as two biomarker proteins. Accordingly, in a sample of saliva from a patient, the concentrations are measured of the proteins described herein. Based on the concentrations as measured, a value is determined reflecting the joint concentrations for said proteins. This value is typically used to calculate the probability of a treatment being successful, and compared with respective threshold values reflecting in the same manner the joint concentrations associated successful treatment of periodontitis. The comparison allows assessing whether the testing value is indicative of successful treatment of periodontitis in said patient.

**Claims**

1. An *in vitro* method for assessing or predicting the response of a human patient to treatment of periodontal disease, wherein the method comprises:

   - detecting, in a sample of saliva from said human patient suffering from periodontal disease, the concentrations of the proteins:

      (i) Hemoglobin subunit delta (Hb-delta) and Pyruvate kinase (PK); or
      (ii) Keratin-4 (K-4) and at least two of Alpha-1-acid glycoprotein (A1AGP), Pyruvate Kinase (PK) and Matrix metalloproteinase-8 (MMP-8); or
      (iii) Alpha-1-acid glycoprotein (A1AGP), Pyruvate Kinase (PK) and S100 calcium binding protein A8 (S100A8);

   - determining at least one testing value reflecting the joint concentrations determined for said proteins;
   - comparing said testing value with a threshold value reflecting in the same manner the joint concentrations associated with successful treatment of periodontal disease, so as to assess whether the testing value is indicative for successful treatment of periodontal disease in said patient.

2. An *in vitro* method according to claim 1, wherein the method assesses the response of a human patient previously diagnosed as having periodontitis and that has received treatment for that periodontitis.

3. An *in vitro* method according to claim 1 or claim 2, wherein the concentrations of the proteins Hemoglobin subunit delta (Hb-delta) and Pyruvate kinase (PK) are detected.

4. An *in vitro* method according to claim 1, wherein the method predicts the response of a human patient to a treatment for periodontitis, optionally wherein the treatment is proposed or was administered no more than 7, 6, 5, 4, 3, 2, or 1 days prior to assessment.

5. An *in vitro* method according to claim 1 or claim 4, wherein the concentrations of the proteins Keratin-4 (K-4) and at least two of Alpha-1-acid glycoprotein (A1AGP), Pyruvate Kinase (PK) and Matrix metalloproteinase-8 (MMP-8) are detected.

6. An *in vitro* method according to claim 1 or claim 4, wherein the concentrations of the proteins Alpha-1-acid glycoprotein (A1AGP), Pyruvate Kinase (PK) and S100 calcium binding protein A8 (S100A8) are detected.

7. A method according to any preceding claim, wherein the age of the subject is determined and the testing value reflects the joint concentrations determined for said proteins in combination with the age of the subject.

8. A method according to any preceding claim, wherein the threshold value is based on the concentration or concentrations determined for the proteins in one or more reference samples each sample associated with the successful treatment of periodontitis or the unsuccessful treatment of periodontitis.

9. A method according to claim 1, wherein the concentrations of the proteins:

   (i) Hemoglobin subunit delta (Hb-delta) and Pyruvate kinase (PK); or
   (ii) Alpha-1-acid glycoprotein (A1AGP), Pyruvate Kinase (PK) and S100 calcium binding protein A8 (S100A8); or
   (iii) Keratin-4 (K-4), Alpha-1-acid glycoprotein (A1AGP), and Pyruvate Kinase (PK); or
   (iv) Keratin-4 (K-4) Alpha-1-acid glycoprotein (A1AGP), and Matrix metalloproteinase-8 (MMP-8); or
   (v) Keratin-4 (K-4), Pyruvate Kinase (PK), and Matrix metalloproteinase-8 (MMP-8); or
   (vi) Keratin-4 (K-4), Alpha-1-acid glycoprotein (A1AGP), Pyruvate Kinase (PK) and Matrix metalloproteinase-8 (MMP-8);

are detected.

10. A method according to any one of the preceding claims, wherein the concentration values determined are arithmetically processed into a number between 0 and 1.

11. The use of the proteins:

(i) Hemoglobin subunit delta (Hb-delta) and Pyruvate kinase (PK); or
(ii) Keratin-4 (K-4) and at least two of Alpha-1-acid glycoprotein (A1AGP), Pyruvate Kinase (PK) and Matrix metalloproteinase-8 (MMP-8); or
(iii) Alpha-1-acid glycoprotein (A1AGP), Pyruvate Kinase (PK) and S100 calcium binding protein A8 (S100A8);

in a sample of saliva of a human patient, as biomarkers for assessing whether the patient will respond, or has responded to, periodontal disease treatment.

12. The use according to claim 11, wherein the age of the human patient is also used as a biomarker.

13. A system for assessing or predicting the response of a human patient to treatment of periodontal disease, the system comprising:

- detection means able and adapted to detect in a sample of saliva of the human patient the proteins:

(i) Hemoglobin subunit delta (Hb-delta) and Pyruvate kinase (PK); or
(ii) Keratin-4 (K-4) and at least two of Alpha-1-acid glycoprotein (A1AGP), Pyruvate Kinase (PK) and Matrix metalloproteinase-8 (MMP-8); or
(iii) Alpha-1-acid glycoprotein (A1AGP), Pyruvate Kinase (PK) and S100 calcium binding protein A8 (S100A8);

- a processor able and adapted to determine from the determined concentrations of said proteins an indication whether the periodontal disease in the patient has been or will be successfully treated.

14. A system according to claim 13, further comprising a container for receiving an oral fluid sample, the container comprising the detection means.

15. A system according to claim 13 or 14, further comprising:

- a user interface for presenting the indication to a user; and
- a data connection between the processor and the user interface for transferring the indication from the processor to the user interface.

16. A system according to any one of claims 13 to 15, wherein the processor is enabled to function by means of an internet-based application.

17. A system according to any of claims 13 to 16, wherein the interface is capable of putting in information on the age of the subject and the processor is able and adapted to determine from the determined concentrations of said proteins, an indication that the patient has been or will be successfully treated.

18. A kit for detecting at least two biomarkers for successful treatment of periodontal disease in a sample of saliva of a human patient, said kit comprising one or more detection reagents for detecting:

(i) Hemoglobin subunit delta (Hb-delta) and Pyruvate kinase (PK); or
(ii) Keratin-4 (K-4) and at least two of Alpha-1-acid glycoprotein (A1AGP), Pyruvate Kinase (PK) and Matrix metalloproteinase-8 (MMP-8); or
(iii) Alpha-1-acid glycoprotein (A1AGP), Pyruvate Kinase (PK) and S100 calcium binding protein A8 (S100A8).

19. A kit according to claim 18, wherein the one or more detecting reagents comprise at least three detection reagents, optionally:

a first detection reagent for detecting Keratin-4 (K-4), a second detection reagent for detecting Alpha-1-acid glycoprotein (A1AGP), and a third detection reagent for detecting Pyruvate Kinase (PK); or

a first detection reagent for detecting Keratin-4 (K-4), a second detection reagent for detecting Alpha-1-acid glycoprotein (A1AGP), and a third detection reagent for detecting Matrix metalloproteinase-8 (MMP-8); or

a first detection reagent for detecting Keratin-4 (K-4), a second detection reagent for detecting Pyruvate Kinase (PK), and a third detection reagent for detecting Matrix metalloproteinase-8 (MMP-8); or

a first detection reagent for detecting Keratin-4 (K-4), a second detection reagent for detecting Alpha-1-acid glycoprotein (A1AGP), a third detection reagent for detecting Pyruvate Kinase (PK) and a fourth detection reagent for detecting Matrix metalloproteinase-8 (MMP-8).

20. A kit according to claim 18 or 19, wherein the one or more detecting reagents are contained on a solid support.

21. A kit according to any one of the claims 18 to 20, wherein the one or more detection reagents consist of detection reagents for:

(i) Hemoglobin subunit delta (Hb-delta) and Pyruvate kinase (PK); or
(ii) Alpha-1-acid glycoprotein (A1AGP), Pyruvate Kinase (PK) and S100 calcium binding protein A8 (S100A8); or
(iii) Keratin-4 (K-4), Alpha-1-acid glycoprotein (A1AGP), and Pyruvate Kinase (PK); or
(iv) Keratin-4 (K-4) Alpha-1-acid glycoprotein (A1AGP), and Matrix metalloproteinase-8 (MMP-8); or
(v) Keratin-4 (K-4), Pyruvate Kinase (PK), and Matrix metalloproteinase-8 (MMP-8); or
(vi) Keratin-4 (K-4), Alpha-1-acid glycoprotein (A1AGP), Pyruvate Kinase (PK) and Matrix metalloproteinase-8 (MMP-8).

22. An *in vitro* method for determining a change in status of periodontal disease due to treatment of the disease, in a human patient over a therapeutic time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the proteins:

(i) Hemoglobin subunit delta (Hb-delta) and Pyruvate kinase (PK); or
(ii) Keratin-4 (K-4) and at least two of Alpha-1-acid glycoprotein (A1AGP), Pyruvate Kinase (PK) and Matrix metalloproteinase-8 (MMP-8); or
(iii) Alpha-1-acid glycoprotein (A1AGP), Pyruvate Kinase (PK) and S100 calcium binding protein A8 (S100A8);

and comparing the concentrations, whereby a difference in any one, two, or three or more of the concentrations, reflects a change in status.

23. A method of determining whether a human patient has been or will be successfully treated for periodontal disease, comprising detecting in a sample of saliva of the human patient the proteins:

(i) Hemoglobin subunit delta (Hb-delta) and Pyruvate kinase (PK); or
(ii) Keratin-4 (K-4) and at least two of Alpha-1-acid glycoprotein (A1AGP), Pyruvate Kinase (PK) and Matrix metalloproteinase-8 (MMP-8); or
(iii) Alpha-1-acid glycoprotein (A1AGP), Pyruvate Kinase (PK) and S100 calcium binding protein A8 (S100A8);

and assessing whether the human patient has been or will be successfully treated for periodontal disease on the basis of the concentrations of said proteins in said sample.

24. A method of detecting the proteins:

(i) Hemoglobin subunit delta (Hb-delta) and Pyruvate kinase (PK); or
(ii) Keratin-4 (K-4) and at least two of Alpha-1-acid glycoprotein (A1AGP), Pyruvate Kinase (PK) and Matrix metalloproteinase-8 (MMP-8); or
(iii) Alpha-1-acid glycoprotein (A1AGP), Pyruvate Kinase (PK) and S100 calcium binding protein A8 (S100A8);

in a human patient, comprising:

(a) obtaining a saliva sample from a human patient; and
(b) detecting whether the proteins are present in the sample by contacting the sample with one or more detecting reagents for binding said proteins and detecting binding between each protein and the one or more detecting reagents.

FIG. 1

Europäisches
Patentamt

European
Patent Office

Office européen
des brevets

**EUROPEAN SEARCH REPORT**

Application Number

EP 18 16 6979

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/037924 A2 (KONINKL PHILIPS NV [NL]) 13 March 2014 (2014-03-13) | 18-21 | INV. G01N33/68 |
| A | * the whole document * | 1-12, 22-24 | |
| | ----- | | |
| X | KR 2011 0135911 A (KYUNGPOOK NAT UNIV IND ACAD [KR]) 20 December 2011 (2011-12-20) | 18-21 | |
| A | * the whole document * | 1-12, 22-24 | |
| | ----- | | |
| X | WIRGINIA KRZYSCIAK ET AL: "Relationship between Pyruvate Kinase Activity and Cariogenic Biofilm Formation in Streptococcus mutans Biotypes in Caries Patients", FRONTIERS IN MICROBIOLOGY, vol. 8, 16 May 2017 (2017-05-16), XP055478296, DOI: 10.3389/fmicb.2017.00856 | 18-21 | |
| A | * the whole document * | 1-12, 22-24 | **TECHNICAL FIELDS SEARCHED (IPC)** |
| | ----- | | |
| X | WO 2015/200898 A1 (RELEVANCE HEALTH [US]; WADE JILL [US]) 30 December 2015 (2015-12-30) | 13-17 | G01N |
| | * the whole document * | | |
| | ----- | | |

~~The present search report has been drawn up for all claims~~

| Place of search | Date of completion of the search | Examiner |
|---|---|---|
| Munich | 29 May 2018 | Stricker, J |

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-24(partially)

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION**
**SHEET B**

Application Number

EP 18 16 6979

The Search Division considers that the present European patent application does not comply with the requirements of unity of invention and relates to several inventions or groups of inventions, namely:

1. claims: 1-24(partially)

An in vitro method for assessing or predicting the response of a human patient to treatment of periodontal disease,
The use of proteins as biomarkers for assessing whether the patient will respond, or has responded to, periodontal disease treatment,
A system for assessing or predicting the response of a human patient to treatment of periodontal disease,
A kit for detecting at least two biomarkers for successful treatment of periodontal disease in a sample of saliva of a human patient,
An in vitro method for determining a change in status of periodontal disease due to treatment of the disease,
A method of determining whether a human patient has been or will be successfully treated for periodontal disease,
A method of detecting proteins;
wherein the following proteins are detected:
(i) Hemoglobin subunit delta (Hb-delta) and Pyruvate kinase (PK).
---

2. claims: 1-24(partially)

The same as "Invention 1", but wherein the proteins are:
(iia) Keratin-4 (K-4), Alpha-1-acid glycoprotein (A1AGP) and Pyruvate Kinase (PK).
---

3. claims: 1-24(partially)

The same as "Invention 1", but wherein the proteins are:
(iib) Keratin-4 (K-4), Alpha-1-acid glycoprotein (A1AGP) and Matrix metalloproteinase-8 (MMP-8).
---

4. claims: 1-24(partially)

The same as "Invention 1", but wherein the proteins are:
(iic) Keratin-4 (K-4), Pyruvate Kinase (PK) and Matrix metalloproteinase-8 (MMP-8).
---

5. claims: 1-24(partially)

The same as "Invention 1", but wherein the proteins are:
(iid) Keratin-4 (K-4), Alpha-1-acid glycoprotein (A1AGP), Pyruvate Kinase (PK) and Matrix metalloproteinase-8 (MMP-8).
---

6. claims: 1-24(partially)

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

```
The same as "Invention 1", but wherein the proteins are:
(iii) Alpha-1-acid glycoprotein (A1AGP), Pyruvate Kinase
(PK) and S100 calcium binding protein A8 (S100A8).
---
```

**ANNEX TO THE EUROPEAN SEARCH REPORT
ON EUROPEAN PATENT APPLICATION NO.**

EP 18 16 6979

This annex lists the patent family members relating to the patent documents cited in the above-mentioned European search report.
The members are as contained in the European Patent Office EDP file on
The European Patent Office is in no way liable for these particulars which are merely given for the purpose of information.

29-05-2018

| Patent document cited in search report | | Publication date | Patent family member(s) | | Publication date |
|---|---|---|---|---|---|
| WO 2014037924 | A2 | 13-03-2014 | BR 112015004881 | A2 | 04-07-2017 |
| | | | CA 2884089 | A1 | 13-03-2014 |
| | | | CN 104620110 | A | 13-05-2015 |
| | | | EP 2893353 | A2 | 15-07-2015 |
| | | | JP 2015529333 | A | 05-10-2015 |
| | | | KR 20150052311 | A | 13-05-2015 |
| | | | RU 2015113089 | A | 27-10-2016 |
| | | | US 2015219665 | A1 | 06-08-2015 |
| | | | WO 2014037924 | A2 | 13-03-2014 |
| KR 20110135911 | A | 20-12-2011 | NONE | | |
| WO 2015200898 | A1 | 30-12-2015 | CA 2952630 | A1 | 30-12-2015 |
| | | | EP 3161481 | A1 | 03-05-2017 |
| | | | US 2017199189 | A1 | 13-07-2017 |
| | | | WO 2015200898 | A1 | 30-12-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RAMSEIER et al.** *J Periodontol.,* March 2009, vol. 80 (3), 436-46